(19) **Europäisches Patentamt / European Patent Office / Office européen des brevets**

(11) **EP 3 617 704 B1**

(12) # EUROPEAN PATENT SPECIFICATION

(45) Date of publication and mention
of the grant of the patent:
**22.07.2026 Bulletin 2026/30**

(21) Application number: **18192036.4**

(22) Date of filing: **31.08.2018**

(51) International Patent Classification (IPC):
**G01N 33/00** *(2006.01)*     **G01N 33/22** *(2006.01)*
**G06F 17/10** *(2006.01)*

(52) Cooperative Patent Classification (CPC):
**G01N 33/225; G01N 33/004; G01N 33/0047;
G06F 17/10**

(54) **METHOD AND APPARATUS FOR ESTIMATING TRANSIT TIMES AND FRACTIONS OF FLUIDS AND USE OF SAME FOR TRACKING FLUIDS IN FLUID DISTRIBUTION GRIDS**

VERFAHREN UND VORRICHTUNG ZUR SCHÄTZUNG VON LAUFZEITEN UND FRAKTIONEN VON FLÜSSIGKEITEN UND DEREN VERWENDUNG ZUR VERFOLGUNG VON FLÜSSIGKEITEN IN FLÜSSIGKEITSVERTEILUNGSNETZEN

PROCÉDÉ ET APPAREIL D'ESTIMATION DE DURÉES DE TRANSIT ET DE FRACTIONS DE FLUIDES ET LEUR UTILISATION POUR LE SUIVI DE FLUIDES DANS DES GRILLES DE DISTRIBUTION DE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.03.2020 Bulletin 2020/10**

(73) Proprietors:
- **Hochschule für angewandte Wissenschaften München**
  80335 München (DE)
- **SmartSim GmbH**
  45131 Essen (DE)

(72) Inventors:
- **Schenk, Joachim**
  81476 München (DE)
- **Athanassios, Alexiou**
  80933 München (DE)
- **Fiebig, Christian**
  44795 Bochum (DE)

(74) Representative: **Maiwald GmbH**
**Elisenhof**
**Elisenstraße 3**
**80335 München (DE)**

(56) References cited:
**EP-A1- 2 450 704**

- **FIEBIG CHRISTIAN ET AL: "Gas quality tracking in gas grids including gas from renewable sources", 1 January 2013 (2013-01-01), XP093273487, Retrieved from the Internet <URL:https://web.archive.org/web/20170603202222/http://www.gerg.eu/public/uploads/files/publications/academic_network/2012/egatec2013_AcademicNetwork.pdf>**
- **BENJAMIN HILLER ET AL: "A system to evaluate gas network capacities: Concepts and implementation", EUROPEAN JOURNAL OF OPERATIONAL RESEARCH, 1 February 2017 (2017-02-01), pages 797 - 808, XP055566257, Retrieved from the Internet <URL:https://www.google.com/url?sa=t&rct=j&q=&esrc=s&source=web&cd=1&cad=rja&uact=8&ved=2ahUKEwj13oy89fDgAhVEJ1AKHU-7AvEQFjAAegQIBxAB&url=https%3A%2F%2Fwww.sciencedirect.com%2Fscience%2Farticle%2Fpii%2FS0377221718301681&usg=AOvVaw2RTDFraSRae6hSFJwD_onm> [retrieved on 20190307], DOI: 10.1016/j.ejor.2018.02.035**
- **MARTIN GROSS ET AL: "On the complexity of instationary gas flows", OPERATIONS RESEARCH LETTERS., vol. 46, no. 3, 17 February 2018 (2018-02-17), NL, pages 286 - 290, XP055566195, ISSN: 0167-6377, DOI: 10.1016/j.orl.2018.01.007**

EP 3 617 704 B1

- **KENNETH L COOKE ET AL: "The shortest route through a network with time-dependent internodal transit times", JOURNAL OF MATHEMATICAL ANALYSIS AND APPLICATIONS, vol. 14, no. 3, 1 June 1966 (1966-06-01), AMSTERDAM, NL, pages 493 - 498, XP055566258, ISSN: 0022-247X, DOI: 10.1016/0022-247X(66)90009-6**

**Description**

FIELD OF THE INVENTION

The present invention relates to a method for tracking fluids, of which comprising trackable fluid properties, in a variety of fluid distribution networks, and more particularly to the estimation of transit times of said fluids, originating at source nodes and use of the same for the estimation of fractions of tracked fluids in mixtures at exit nodes using signal processing techniques.

BACKGROUND OF THE INVENTION

Benjamin Hiller ET AL: "A system to evaluate gas network capacities: Concepts and implementation", European Journal of Operational Research, 1 February 2017 (201 7 -02-0 1 ), pages 7 97 -808, XP055566257, describes a mathematical optimization project that aims to develop a new set of tools for the evaluation of gas network capacities. The objectives and challenges as they have arisen in the project are described, as well as the developments and design decisions that have been made to meet the requirements.
MARTIN GROSS ET AL: "On the complexity of instationary gas flows", OPERATIONS RESEARCH LETTERS, vol. 46, no. 3, 17 February 2018 (2018-02-17), pages 286-290, XP0555661 95 describes a simplified model of transient gas flows, which is built up from a sequence of k stationary gas flows.
KENNETH L COOKE ET AL: "The shortest route through a network with time-dependent internodal transit times", JOURNAL OF MATHEMATICAL ANALYSIS AND APPLICATIONS, vol. 14, no. 3, 1 June 1966 (1966-06-01), pages 493-498, XP055566258, describes a mathematical optimization project that describes a modified form of Bellman's iteration scheme for determining the shortest route between any two nodes in a network. It converges to the shortest route between any two nodes in a finite number of iterations and for any starting time, provided that certain initial conditions are met.
In the industry, e.g. the chemical industry, in medical applications, in the field of microfluidics and in particular in the distribution of energy (e.g. gas), there is an increasing need of tracking fluids from source nodes to exit or consumption nodes, e.g. for the purpose of process monitoring or for customer invoicing. Natural gas distribution networks for instance, are used to transport large amounts of energy. In the past, natural gas with typically insignificantly varying energy content, i.e. almost constant calorific value, was injected into distribution networks. Today transported gases are more likely to be of different composition and therefore of different energy content. This is due to the fact that gases of different origin, e.g. hydrogen from Power2Gas plants, bio natural gas from bio natural gas plants, general substitute natural gas (SNG) or liquefied natural gas (LNG) from the Middle East are injected concurrently into natural gas distribution grids, as described by Altfeld K., Schley P., "Entwicklung der Erdgasbeschaffenheiten in Europa", 2012, Fachbericht Gaswärme International 2-2012, 57 - 63.
Due to the increasing fluctuations of the calorific value, the invoicing of end customers becomes problematic. The invoicing of end customers is regulated in DVGW worksheet G685: The calorific values of different feed-in points may not differ by more than 2% from the volume-weighted invoicing calorific value of the grid area as described by DVGW-Arbeitsblatt G 685, "Gasabrechnung," DVGW, 2008.
For instance, in the case of bio methane, which is fed into H-gas grids, this means that the raw biogas must be conditioned to the calorific value of the prevailing natural gas in the grid by adding LPG. This results in immense costs per system, estimated in the article by Schenk J., P. Schley, A. Hielscher, C. Fernandez und S. Maurer, "Brennwertverfolgung in Verteilnetzen: Teil 2: Auswertung Feldversuch," gwf-Gas/Erdgas, pp. 676-683, 2011., at around € 15,000 per year per 100 $m^3$ / h of biogas feed-in capacity.
As an alternative to the calorific value adjustment, G 685 also offers the possibility of calorific value allocation. An end customer can be billed with his own, weighted calorific value if this calorific value can be assigned to the calibrated calorific values at the feed-in points.
This assignment is extremely complex and has only recently become state of the art. The SmartSim method as described by the article P. Schley, J. Schenk und A. Hielscher, "Brennwertverfolgung in Verteilnetzen: Teil 1: Entwicklung und Validierung des Verfahrens," gwf-Gas/Erdgas, pp. 552-556, 2011., is a representative of computational gas and calorific value tracking systems and the underlying method is protected by EP 2 450 704 B1.
A challenge in the use of calorific value allocation methods, analogous to the SmartSim process, is the validation of the method. An insight into the elaborate validation by means of measurement data, which is usually collected with a mobile process gas chromatograph (PGC), is available by Schenk J., P. Schley, A. Hielscher, C. Fernandez und S. Mäurer, "Brennwertverfolgung in Verteilnetzen: Teil 2: Auswertung Feldversuch," gwf-Gas/Erdgas, pp. 676-683, 2011. In particular, it is currently not possible to directly validate the gas transit times and gas fractions from the feed-in points to the exit points.
In order to simplify the validation, the article by C. Fiebig, A. Hilscher, R. Span, P. Schley und J. Schenk, "Gas Quality Tracking in Gas Grids including Gases from Renewable Sources", Kopenhagen: EGATEC, 2013, proposes to record data proportional to gas species (for example the $CO_2$, methane, etc. content) with simple infrared sensors at feed-in and feed-out points and to determine the transit times from the signal trajectories of the species. The therein introduced methods are based on a time-invariant signal propagation model.

SUMMARY OF THE INVENTION

The foregoing and other objects are solved by the subject-matter of the present invention.

According to a first aspect of the present invention, a method is provided, used for the estimation of transit times $\tau_{o,q}(t)$ of fluids between a first number $Q \geq 1$ of upstream network nodes $q = 1,2, ... , Q$, interconnected with a second number $O \geq 1$ of downstream network nodes $o = 1,2, ... , O$, and corresponding fractions $\alpha_{o,q}(t)$ of the fluids, sampled at each upstream network node of said first number $Q$ of the upstream network nodes, composing the fluids sampled at each downstream node of said second number $O$ of the downstream network nodes, in at least one part of a fluid distribution network.

The method used for determining the transit times and the fractions comprises the step of using at least one sensor element operated to provide sampled upstream data $s_{q,e}^{Q}(t)$ related to a number $E \geq 1$ of fluid properties $e = 1,2, ... , E$ from the fluids of said first number $Q$ of the upstream network nodes $q = 1,2, ..., Q$ and at least one sensor element operated to provide sampled downstream data $s_{o,e}^{O}(t)$ related to said number $E$ of the fluid properties e from the fluids of at least one downstream network node of said second number $O$ of the downstream network nodes $o$.

The method for determining the transit times and the fractions further comprises the step of iteratively utilizing a finite state machine for the alignment between individual measurements of said sampled upstream data $s_{q,e}^{Q}(t)$, derived by sampling upstream network nodes $q$ at discrete points in time t to related individual points in time $t + \tau_{o,q}(t)$ of said sampled downstream data $s_{o,e}^{O}(t)$ related to the fluid properties e, derived by sampling at least one of the downstream network nodes of said number $O$.

In other words, the identical fluid properties e are provided by the sampled downstream data $s_{o,e}^{O}(t)$ as well as by the sampled upstream data $s_{q,e}^{Q}(t)$, e.g. if three specific fluid properties are sampled as part of the upstream data $s_{q,e}^{Q}(t)$ the very same three specific fluid properties are sampled as part of the sampled downstream data $s_{o,e}^{O}(t)$.

According to one embodiment of the present invention, the method further comprises the step of utilizing said finite state machine to minimize a total error sum of alignment errors along a path $\omega$ defined by pairings of time indexes ($t, t + \tau_{o,q}(t)$) corresponding to states $\sigma(t, t + \tau_{o,q}(t))$ of individual causal alignments of said sampled upstream data $s_{q,e}^{Q}(t)$ at sample time $t$ and downstream data $s_{o,e}^{O}(t + \tau_{o,q}(t))$ at sample time t + $\tau_{o,q}(t)$, wherein aligned upstream and downstream node data are related to at least one of said number $E$ of fluid properties $e$.

According to one embodiment of the present invention, the fluid properties e comprise at least one of the following - in other words, at least one of the following group consisting of: a concentration of countable fluid particles, a concentration of hydrogen, a concentration of carbon dioxide, a concentration of methane, a concentration of nitrogen, a concentration of argon, a concentration of water, a concentration of helium, a concentration of ethane, a concentration of propane, a concentration of butane, a concentration of carbon oxide, a concentration of an odorant, a concentration of an alkane, a concentration of an alkene, a concentration of oxygen, a concentration of hydrocarbons, a concentration of sulphur, a concentration of hydrogen sulfide, a calorific value, a normal density, a Wobbe index and combinations thereof.

The fluid properties e may for instance be measured using infrared sensors, or using mobile process gas chromatographs or using a sensor for measuring a thermal conductivity, or gravimetric and paramagnetic effects. The sensor used for measuring the fluid properties e may for example be a sensor configured to measure a mid-Infrared absorption or using optical techniques such as non dispersive infrared, NDIR, or photo-acoustic methods or a gas flow meter.

According to one embodiment of the present invention, said sampled downstream data $s_{o,e}^{O}(t)$ related to the $E \geq 1$ fluid properties is derived through sampling of fluids at successive points in time at at least one downstream node o and said sampled upstream data $s_{q,e}^{Q}(t)$ related to the $E \geq 1$ fluid properties is derived through sampling of fluids at successive points in time at at least one of said upstream network nodes $q$ contributing to the gas composition at the at least one downstream network node of the downstream network nodes $o$.

According to another embodiment of the present invention, said sampled downstream data $s_{o,e}^{O}(t)$ and/or said sampled upstream data $s_{q,e}^{Q}(t)$ related to the $E \geq 1$ fluid properties is derived through sampling of fluids with constant or varying sampling frequency at any sensor location in the fluid distribution network.

According to a further embodiment of the present invention, physical constraints, expressed as a global lower bound transit time $\tau_{o,q,\min}$ and a global upper bound transit time $\tau_{o,q,\max}$, with $\tau_{o,q,\min} \leq \tau_{o,q}(t) \leq \tau_{o,q,\max}$, are taken into account for the estimation of the transit times $\tau_{o,q}(t)$ and the fractions $\alpha_{o,q}(t)$, for at least one specific interconnected pair of nodes $o, q$.

According to one embodiment of the present invention, the global lower bound transit times $\tau_{o,q,\min}$ and the global upper bound transit times $\tau_{o,q,\max}$ are defined individually for all, with the method according to the first aspect, processed interconnected pairs of nodes $o, q$.

According to another embodiment of the present invention, the global lower bound transit times $\tau_{o,q,\min}$ and the global upper bound transit times $\tau_{o,q,\max}$ are determined for at least one specific interconnected pair of nodes $o, q$ through optimization.

According to still another embodiment of the present invention, local lower bound transit times $\tau_{o,q,\min}(t)$ and local upper bound transit times $\tau_{o,q,\max}(t)$ are defined for at least one specific interconnected pair of nodes $o, q$.

According to still another embodiment of the present invention, the local lower bound transit times $\tau_{o,q,\min}(t)$ and the local upper bound transit times $\tau_{o,q,\max}(t)$ are defined for all processed specific interconnected pairs of nodes $o, q$.

According to an additional embodiment of the present invention, the local lower bound transit times $\tau_{o,q,\min}(t)$ and the local upper bound transit times $\tau_{o,q,\max}(t)$ are determined for a at least one specific interconnected pair of nodes $o, q$ through optimization. According to a further embodiment of the present invention, the optimization is performed using at least one similarity measure between the downstream data and the upstream data.

According to a further embodiment of the present invention, said sampled upstream data $s_{q,e}^{Q}(t)$ is sampled at a number of at least $Q \geq 2$ upstream nodes and said sampled downstream data $s_{o,e}^{O}(t)$ sampled at a number of least $\overline{O} \geq 1$ network downstream nodes.

According to a further embodiment of the present invention, said sampled downstream data $s_{o,e}^{O}(t)$ and/or said sampled upstream data $s_{q,e}^{Q}(t)$ is related to $E \geq 2$ fluid properties, wherein at least one of the $E$ fluid properties is processed with said finite state machine.

According to an embodiment of the present invention, the fluid transported in the distribution network is combustible gas and the fluid distribution network denotes gas distribution networks.

According to a second aspect of the present invention, a computer program product is provided comprising computer-readable instructions which, when loaded and executed on processor, performs the method according to any one of the embodiments of the first aspect or the first aspect as such.

According to a third aspect of the present invention, an apparatus is provided, used for the estimation of transit times $\tau_{o,q}(t)$ of fluids between a first number $Q \geq 1$ of upstream network nodes $q = 1,2, ... , Q$, interconnected with a second number $O \geq 1$ of downstream network nodes $o = 1,2, ... , O$, and corresponding fractions $\alpha_{o,q}(t)$ of the fluids, sampled at each upstream network node of said first number $Q$ of the upstream network nodes, composing the fluids sampled at each downstream node of said second number 0 of the downstream network nodes, in at least one part of a fluid distribution network.

The apparatus comprises at least one sensor element operated to provide sampled upstream data $s_{q,e}^{Q}(t)$ related to a number $E \geq 1$ of fluid properties $e = 1,2, ..., E$ from the fluids of said first number $Q$ of the upstream network nodes $q = 1,2, ..., Q$ and at least one sensor element operated to provide sampled downstream data $s_{o,e}^{O}(t)$ related to said number $E$ of the fluid properties $e$ from the fluids of at least one downstream network node of said second number $O$ of the downstream network nodes $o$.

The apparatus further comprises a processor configured to iteratively utilize a finite state machine for the alignment between individual measurements of said sampled upstream data $s_{q,e}^{Q}(t)$, derived by sampling upstream network nodes $q$ at discrete points in time t to related individual points in time $t + \tau_{o,q}(t)$ of said sampled downstream data $s_{o,e}^{O}(t)$ related to the identical fluid properties e, derived by sampling at least one of the downstream network nodes of said number $O$.

A computer program performing the method of the present invention may be stored on a computer-readable medium. A computer-readable medium may be a floppy disk, a hard disk, a CD, a DVD, an USB (Universal Serial Bus) storage device, a RAM (Random Access Memory), a ROM (Read Only Memory) and an EPROM (Erasable Programmable Read Only Memory).

A computer-readable medium may also be a data communication network, for example the Internet, which allows downloading a program code.

The methods, systems and devices described herein may be implemented as software in a Digital Signal Processor, DSP, in a micro-controller or in any other side-processor or as hardware circuit within an application specific integrated circuit, ASIC, CPLD or FPGA.

The present invention can be implemented in digital electronic circuitry, or in computer hardware, firmware, software, or in combinations thereof, e.g. in available hardware of conventional mobile devices or in new hardware dedicated for processing the methods described herein.

BRIEF DESCRIPTION OF THE DRAWINGS

A more complete appreciation of the invention and the attendant advantages thereof will be more clearly understood by reference to the following schematic drawings, which are not to scale, wherein:

Fig. 1 shows schematic diagrams of a selection of some common network topologies; star (left), tree (center) and meshed (right), illustrated in idealized form according to an exemplary embodiment of the invention;

Fig. 2 shows a mesh, exemplified by a star structure, which is sampled at four nodes, one sink (center) and three sources according to an exemplary embodiment of the invention;

Fig. 3 shows the same network as in Fig. 2 according to an exemplary embodiment of the invention;

Fig. 4 shows the network regarded as a signal-carrying transport system according to an exemplary embodiment of the invention;

Fig. 5 shows a schematic diagram of an illustration of a finite state machine according to an exemplary embodiment of the invention;

Fig. 6 shows a trellis diagram created by rolling out the finite state machine in Fig. 5 over time according to an exemplary embodiment of the invention;

Fig. 7 shows data derived with present invention in comparison to measured data;

Fig. 8 shows data derived with methods according to the state of the art (SmartSim simulations) in comparison to measured data;

Fig. 9 shows a schematic diagram of an apparatus for estimating transit times $\tau_{o,q}(t)$ of fluids and fractions $\alpha_{o,q}(t)$ of the fluids according to an exemplary embodiment of the invention;

Fig. 10 shows a schematic diagram of an apparatus for estimating transit times $\tau_{o,q}(t)$ of fluids and fractions $\alpha_{o,q}(t)$ of the fluids according to an exemplary embodiment of the invention.

DETAILED DESCRIPTION OF EMBODIMENTS

The illustration in the drawings is schematically and not to scale. In different drawings, similar or identical elements are provided with the same reference numerals. Generally, identical parts, units, entities or steps are provided with the same reference symbols in the figures.

The term "upstream network node" as used by the present invention may refer to a source, for instance modelled in analogy to a data source, treating the gas transport as data transmissions in the field of signal processing.

The term "downstream network node" as used by the present invention may refer to a sink, for instance modelled in analogy to a data sink, treating the gas transport as data transmissions in the field of signal processing.

The term "finite state machine" as used by the present invention may refer to a numerical model of computation based on an abstract machine that can be in exactly one of a finite number of states at any given time.

The description of the present invention uses the following notation as a system of symbolic representations:

For sampling points at each source $q$ located upstream sampled at time $t$:

$$s_{q,e}^{Q}(t)$$

(continued)

| | |
|---|---|
| For sampling points at each sink $o$ located downstream sampled at time $t$ | $s_{o,e}^{\text{O}}(t)$ |
| For the source's $q$ contribution (i.e. fraction) at sink $o$ at time $k$: | $\alpha_{o,q}(k)$ |
| For transit times from source $q$ to sink $o$ at time $k$: | $\tau_{o,q}(k)$ |
| For a maximum transit time from source $q$ to sink $o$: | $\tau_{o,q,\text{max}}$ |
| For a minimum transit time from source $q$ to sink $o$: | $\tau_{o,q,\text{min}}$ |

The gas properties in their entirety are denoted by $E$, a realization of this with $e$.

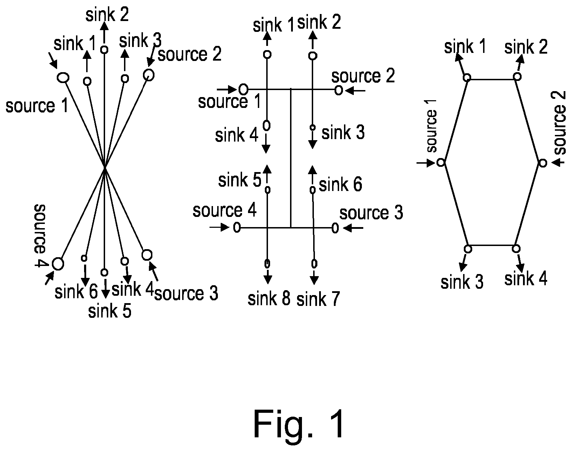

Fig. 1 shows schematic diagrams of a selection of some common network topologies; star (left), tree (center) and meshed (right), illustrated in idealized form.

In the following section, the terms and the structure of the considered networks are briefly presented. This is not a delineation of the applicability of the process.

A (gas) network comprises at least $Q$ feeders, hereafter called "sources", each source being distinguishable, i.e. $q = 1, 2, ..., Q$. Sources feed gas of different compositions into the network. The gases are transported via pipes to the $O$ exit sources, hereafter "sinks", each sink being distinguishable, i.e. $o = 1, 2, ..., O$. Meaningful networks comprise at least one source and one sink, i.e. it is at least $Q = O = 1$. The gas or fluid networks may have different topologies, e.g. they are star-shaped as shown in Fig. 1, left panel, tree-shaped as shown in Fig. 1, center panel, or meshed as shown in Fig. 1, right panel, or appear as any combination of the aforementioned structures.

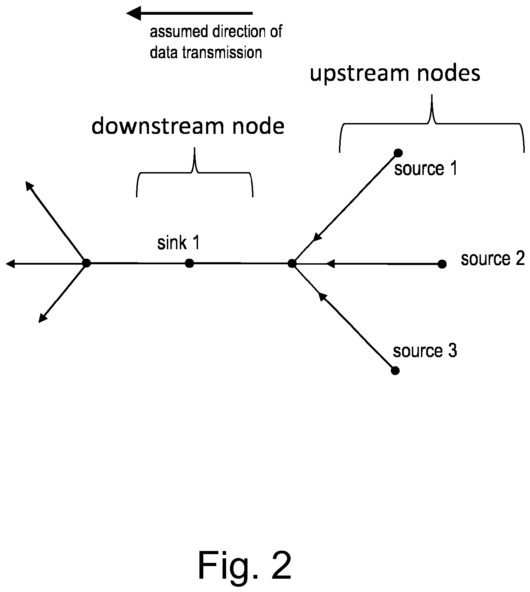

Fig. 2 shows a mesh, exemplified by a star structure, which is sampled at four nodes, one sink provided in the center and three sources. The three node pairings $(o_1, q_1)$, $(o_1, q_2)$ and $(o_1, q_3)$ resulting from the sink and the three sources can be processed by the method presented herein, provided that all measurement data are available. Here, fractions and transit times of the gases from the upstream nodes, commonly referred to as sources, to the downstream nodes, commonly referred to as sinks, are estimated. To do this, one must first set a data transfer direction (arrows) from the sources to the sink.

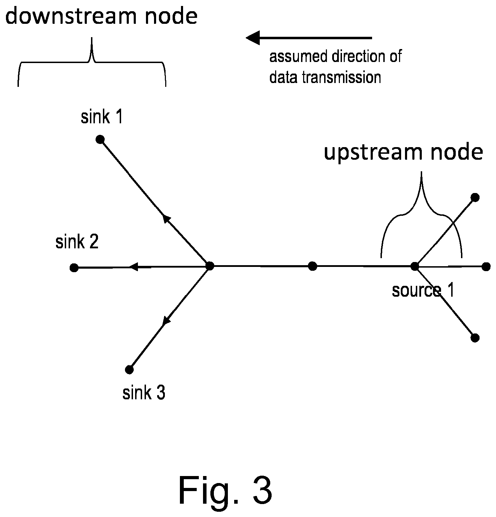

In fluid dynamic distribution networks, due to the possibility of defining a direction of data transmission of the sampled quantities (or properties) along the network lanes, nodes can be accordingly denoted upstream nodes, i.e. sources, as well as downstream nodes, i.e. sinks, as shown in Fig. 2 and 3.

The nodes $o$ and $q$ defined for the method do not have to coincide with the actually existing sources and sinks at the considered network structure. A data transmission direction is defined between every two nodes, so that upstream nodes as sources $q$ and downstream nodes in the transmission direction can be identified as sinks $o$. Nodes can accordingly represent any measurement locations along the distribution network, at which measurement data can also be collected which can be sensibly processed using the method described herein. Only if the direction is correctly specified, transit times and fractions can be determined within the boundary conditions defined below.

Fig. 3 shows the same network as in Fig. 2. However, the approach as shown in Fig. 3 defines a source that was previously processed or modelled as a sink. The node pairings $(o, q)$, with $(o_1, q_1)$, $(o_2, q_1)$, and $(o_3, q_1)$, which result from the presently used or modelled constellation of sensors installed along the network, with three sinks and one source, can also be processed with the method according to the present invention.

However, it is important to know that the method presented herein does not only work with the measurement data of the sources and sinks that are physically present within the considered network. Nodes can be defined anywhere in the network. Only the assumed data transmission directions, indicated by arrows, provide orientations, with the help of which two nodes can be used to define sources and sinks. The prerequisite is that data can be collected at the nodes and that the assumed data transmission direction is correct, which can be switched if needed. Also, alternatively, the use of the terms "data source" $q$, and "data sink" $o$ is allowed.

For example, the source (center of the image) defined by the example in Fig. 3 can be processed as a sink in a previous step as shown in Fig. 2. Data from the upstream sources as shown in Fig. 2 is transported with time delays and data of different sources superimposing each other arbitrarily. In a subsequent step, the estimates derived with the method according to the present invention herein is taken to derive the estimates of the transit times of the data to subsequent downstream nodes.

This approach can be applied arbitrarily to nodes that are in a causal relationship. Of course, it is equivalently possible to process the data sampled at physical source nodes, from one end to the other of the distribution structure, i.e. to the physical sink nodes. However, it may be necessary to collect data at additional critical intermediate points in order to achieve higher accuracy, e.g. in case many sources are superimposing at single sinks in a time-variant manner.

For the method, it is assumed that at each source node $q$ a total of $E$ different properties $e$ of the fluids are time-discretely sampled and recorded in steps $\Delta t$ ($\Delta t$ may vary over time, in other words the steps are at necessarily periodic time intervals). The result is thus given by time-discrete sample points, wherein the index $j$ at the sampling time t and j+1 corresponds to the sampling time $t + \Delta t$. This results in that at each source $q$ a sequence of (vectorial) sampling points is

recorded:

$$\mathbf{s}_q^{\mathrm{Q}}(j) = \left(s_{,1}^{\mathrm{Q}}(j), \ldots, s_{q,E}^{\mathrm{Q}}(j)\right)^{\mathrm{T}} \tag{1}$$

with $s_{q,e}^{\mathrm{Q}}(j)$ being the scalar realization of the property e to the time index j at the source q.

In order to use the method described herein, i.e. according to any embodiment of the present invention, the same properties are also recorded at at least one further node, usually at one of the sinks o, equivalently sampled in a time-discrete manner. The result is given again by further time-discrete samples, the index k being the sampling time t and k + 1 being the sampling time t + Δt. For each sample time index k, the sampled point (vectorially written) for each sampled sink o is derived to

$$\mathbf{s}_o^{\mathrm{O}}(k) = \left(s_{o,e}^{\mathrm{O}}(k), \ldots, s_{o,E}^{\mathrm{O}}(k)\right)^{\mathrm{T}} \tag{2}$$

with $s_{o,e}^{\mathrm{O}}(k)$ being the (scalar) realization of the property e to the time index k at the sink o. However, it is not necessary to sample all sinks of the relevant network. It is therefore quite sufficient to sample only one sink, and also to sample at just one single source. The data generated by the method will then relate to the actual pair of sink and source.

At sinks o, a mixed fluid is usually sampled, which is composed of different fractions of time-varying delayed source fluids (if the gas or fluid network has more than one source). The task of calorific value assignment is to determine the transit times and fractions for at least one node pair of source and sink.

For each sample time index k along the vector $\cdot\, \mathbf{s}_o^{\mathrm{O}}(k)$ of the measured values of the property e of the sinks o, the transit times $\tau_{o,q}(k)$ and fractions $\alpha_{o,q}(k)$ of the sample points of the sources q involved in the composition of the mixed gas is estimated. If the transit times and fractions are known, the (vectorial) estimated value

$$\hat{\mathbf{s}}_o^{\mathrm{O}}(k) = \left(\hat{s}_{o,1}^{\mathrm{O}}(k), \ldots, \hat{s}_{o,E}^{\mathrm{O}}(k)\right)^{\mathrm{T}}, \tag{3}$$

with $\hat{s}_{o,e}^{\mathrm{O}}(k)$ being the (scalar) estimate of the property e at the time index k at the sink o, can be analytically derived to

$$\hat{\mathbf{s}}_o^{\mathrm{O}}(k) = \sum_{q=1}^{Q} \alpha_{o,q}(k) \cdot \mathbf{s}_q^{\mathrm{Q}}\left(k - \tau_{o,q}(k)\right). \tag{4}$$

The transit times of the gases from the individual sources to the sinks and the fractions of the sources for the formation of the mixed gas vary (in some cases very strongly). The scanned properties are to be regarded as time-dependent. The transit times and fractions of the source gases to the sinks are therefore also time-dependent.

The fluctuation of the fractions of the source gases is in the range from 0 to 100%. It is thus possible that for individual sample times, for signal segments, or even continuously over all measurements, only one upstream node delivers 100% of the gas observed at the downstream node. It is also possible that more than one or even all upstream nodes deliver gas to just one downstream node for individual sampling instants, for signal sections or continuously over all measurements. However, for the formation of the estimate from equation (4), the following, physically motivated, boundary conditions should be considered:

At first, $0 \leq \alpha_{o,q}(k) \leq 1$, for all o, q and k: The individual parts are always positive, i.e. there are no "virtual sources" that provide a negative contribution. However, $\alpha_{o,q}(k) = 0$ is explicitly included, i.e. there are sources q which, if necessary, have no influence on the formation of the mixed gas at the current sink, as mentioned above. In addition, $\alpha_{o,q}(k) = 1$ may also hold, i.e. the sink o is fed, if necessary in sections, from only a single source q.

Secondly, $\sum_{q=1}^{Q} \alpha_{o,q}(k) = 1$, it is assumed that from the available data of the sources, the data points of the sink can be completely reconstructed, for instance for at least all required sources.

Thirdly, $(k - \tau_{o,q}(k)) \geq 0$ and $0 \leq \tau_{o,q}(k) \leq k$, with $k \geq 0$, , i.e. the system is causal with regard to the outfeed and the running times are always positive (gas can only be withdrawn if it has been previously fed in).

The task of a calorific value allocation system is therefore to determine the causal transit times $\tau_{o,q}(k)$ and positive fractions $\alpha_{o,q}(k)$ of the source gases q to the mixed gas of the sink o from equation (4) as accurately as possible. Especially when applying calorific value allocation systems such as SmartSim (see below), it has been shown that at each time index *k* for each source *q* time-varying fractions and transit times contribute to form the gas observed at the sinks *o*, i.e. the modelled fluid or gas flows through or in distribution networks are highly time-variant.

The calorific value classification is currently derived - with trivial exceptions, namely grids with only one source, - by computation-based calorific value allocation systems. One realization of a calorific value allocation system is the so-called "SmartSim" method. Here, the determination of the transit times $\tau_{o,q}(k)$ and fractions $\alpha_{o,q}(k)$ from equation (4) are estimated by means of a fluid dynamic simulation of the respective network. The therein used input data thus represents all calibrated feed volumes, feed pressures, topology data such as tube lengths, diameters and roughnesses, calibrated outfeed volumes and estimates for all other exit volumes using standard load profiles. From these input data, volumetric flows and flow velocities within the tubes are determined using standard hydraulic calculation techniques.

Subsequently, the transit times $\tau_{o,q}(k)$ and fractions $\alpha_{o,q}(k)$ are calculated from the flow velocities and the volumetric flows and thus the respectively required gas properties (in each case the calorific value, but usually also the $CO_2$ fraction, the standard density and the $H_2$ content) according to equation (4).

The accuracy of the method, i.e. the deviation of the associated gas properties from the true measured value depends on the accuracy of determining the transit times $\tau_{o,q}(k)$ and fractions $\alpha_{o,q}(k)$. These are determined indirectly by field tests as part of the approval of the SmartSim procedure for each network. During these field tests, the relevant gas properties at the sources and at one (or more) sink(s) are measured by means of PGCs and the qualitative course between measurement and calculation is evaluated.

The input variables that are used in conventional calorific value allocation systems largely determine the maximum possible calorific value reconstruction accuracy. However, the input variables for simulations of gas distribution networks are often faulty: pipe lengths, diameters and roughnesses may not be correctly stored in the corresponding geographic information systems of the network operators, the volume measurements are subject to the traffic error limit and thus have measurement uncertainties.

In addition, the volumes at the exit points (with the exception of customers with recorded demand measurement, so-called "RDM customers") are determined using standard load profiles. These can have errors of over 50%. Especially in meshed systems as shown for instance in Fig. 1, right, these uncertainties in the processed input variables lead to incorrect assignments of the calorific value.

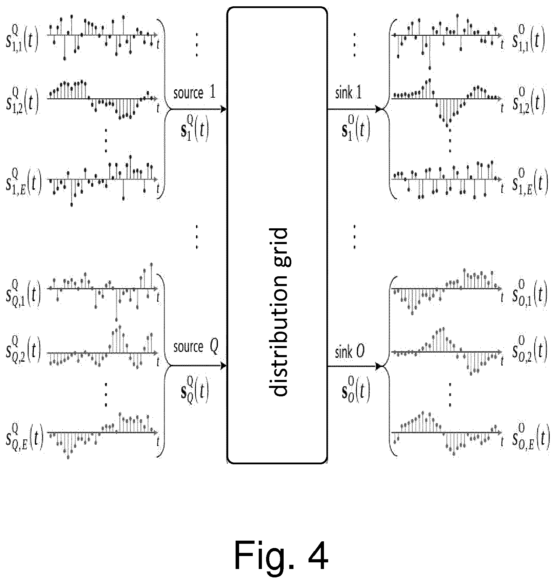

The method presented by the present invention takes a different route for the assignment of gas properties. Any measurable property available after sampling and recording as discrete-time signal for upstream and downstream nodes is considered to be information transported through a network (e.g., a gas distribution network) from a data source to a data sink. The physical gas transport system can therefore also be understood as a signal transmitting system, as illustrated in Fig. 4.

At one or more sources, one or more characteristics (e.g., measurement data proportional to the $CO_2$ or methane or other gaseous components in the gas) are measured. Several properties can also be sampled in parallel. This produces vectors $\mathbf{s}_q^Q(k)$ (see equation (1)), which represent time-discrete measured values of the sampled properties at the sources. Additionally, the same properties are sampled at one (or more) sinks. The sample points of the vector $\mathbf{s}_o^O(k)$ arise as defined by equation (2).

The transit times $\tau_{o,q}(k)$ and fractions $\alpha_{o,q}(k)$ are estimated by means of signal processing from the sampled signals $\mathbf{s}_q^Q(k)$ and $\mathbf{s}_o^O(k)$. The found fractions and transit times may then be used, e.g. for validating the transit times and fractions of a calorific value allocation system or by applying equation (4) to directly calculate a calorific value for customer invoicing.

The present invention thus relates to a method for estimating transit times $\tau_{o,q}(k)$ and fractions $\alpha_{o,q}(k)$ of the gases from the sources to the sinks according to equation (4), using methods of signal processing.

Fig. 4 shows the network regarded as a signal-carrying transport system, via which data $s_{q,e}^Q(k)$ of the sources q is transferred time-variably to the sinks *o*. The signal data $s_{o,e}^O(k)$ at a sink o are sampled to the identical properties e. It is assumed that the system is additive, linear and time-variant.

Hereinafter, the novel method for determining the transit times $\tau_{o,q}(k)$ and fractions $\alpha_{o,q}(k)$ from equation (4) will be described. It can be divided into the following parts:

- Determining the transit times $\tau_{o,q}(k)$ for each source *q* with respect to the sink *o*.
- Determining the fractions $\alpha_{o,q}(k)$ for each source *q* with respect to the sink *o*.

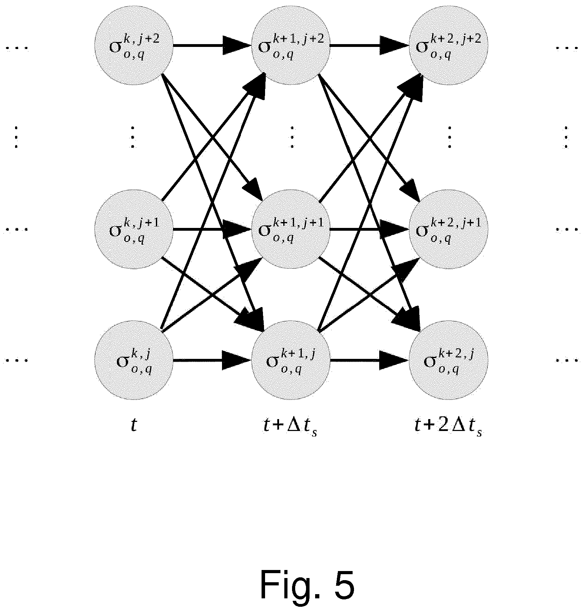

As mentioned, the transmission system of Fig. 4 (as such, a gas transport network, which is considered hereinafter) is highly time-variant, i.e. the transit times of the gases from the sources to the currently considered sink as well as the fractions of sources of mixed gas may vary in each time step. To determine the transit times, therefore, the assignment of a sample point $\mathbf{s}_q^Q(j)$ of the source q to the sample point $\mathbf{s}_o^O(k)$ of the sink o is described by means of a finite state machine. The basic structure of a finite state machine suitable for the approach is shown in Fig. 5.

Fig. 5 shows an illustration of a finite state machine according to an exemplary embodiment of the invention, as it can be used for the compensation of the time variance of the considered transmission system. It shows the definable states $\sigma$ (circles) and the possible transitions (arrows).

In the finite state machine of Fig. 5, the state means

$$\sigma_{o,k}^{q,j} : \qquad \text{the sample point } \mathbf{s}_q^Q(j_i) \text{ of the source is assigned to the sample point } \mathbf{s}_o^O(k) \text{ of the sink."}$$

and further for any transitions between two states

$$\sigma_{o,k}^{q,j_1} \to \sigma_{o,k}^{q,j_2} : \qquad \text{the sample point } \mathbf{s}_q^Q(j_1) \text{ of the source is assigned to the sample point } \mathbf{s}_o^O(k) \text{ of the sink}$$

$$\text{and subsequently the sample point } \mathbf{s}_q^Q(j_2) \text{ of the source is assigned to the sample point}$$

$$\mathbf{s}_o^O(k+1) \text{ of the sink.}$$

Since both $j_1 < j_2$ and $j_1 = j_2$ and even $j_2 > j_1$ may apply, the time-variant flow situations (slowed down flowing gas, stagnant gas, and possibly short-term flow reversal) of the gas transport network are taken into account, and compensated for. If the state $\sigma_{o,k}^{q,j}$ is known, the transit times yield:

$$\tau_{o,q}(k) = j - k, \qquad\qquad\qquad (5)$$

wherein o, q, k and j are taken from the state $\sigma_{o,k}^{q,j}$.

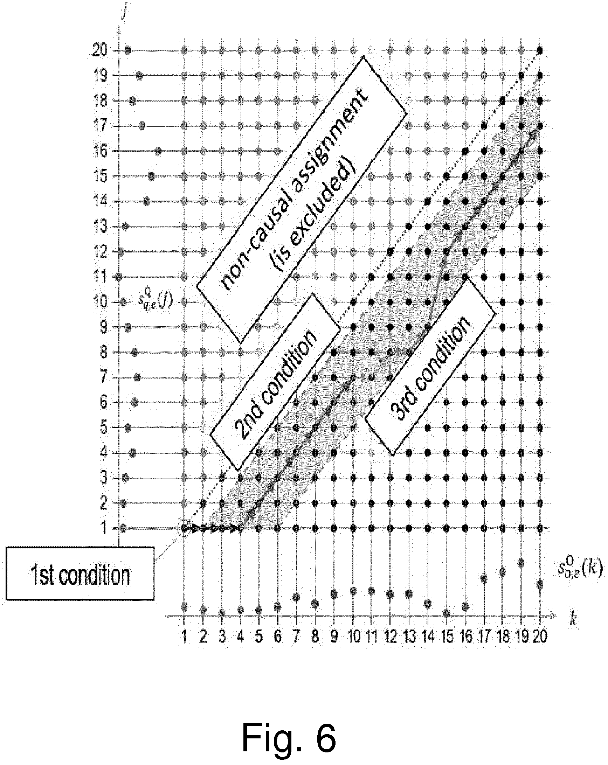

In the following, the optimal state sequence is determined. For this purpose, a temporal roll-out of the finite state machine as shown in Fig. 5 is performed. This results in a trellis diagram, which is shown by the example of Fig. 6.

Fig. 6 shows a trellis diagram created by rolling out the finite state machine of Fig. 5 over time. The three conditions - as provided in more detail below - regarding the physical limitations of the system are also illustrated.

Any conceivable assignment of the source sample points $\mathbf{s}_q^Q(j), \ j = 1, \dots, J$ to all $\mathbf{s}_o^O(k), k = 1, \dots, K$ (i.e. a complete assignment of all $K$ sample points at the feed-out point) corresponds to one path $\omega_o^q(k), k = 1, \dots, K$ through the trellis. The state assignments as described above can also be described by path assignments

$$\sigma_{o,k}^{q,j}: \qquad \omega_o^q(k) = j$$

and

$$\sigma_{o,k}^{q,j_1} \to \sigma_{o,k+1}^{q,j_2}: \qquad \omega_o^q(k) = j_1 \text{ and } \omega_o^q(k+1) = j_2$$

All physical network properties, in particular the requirements for the causality defined above, can be modelled by a restriction regarding valid paths through the trellis. In detail (these are also highlighted in Fig. 6) this means:

i) The assignment of the individual sample points from two vectors $\mathbf{s}_q^Q(j)$ and $\mathbf{s}_o^O(k)$ to each other always begins with the state $\sigma_{o,1}^{q,1}$.

ii) In order to maintain causality, $\tau_{o,q}(k) \geq \tau_{o,q,\min}(k)$, as depicted by the dashed line in the upper part of the grey area of Fig. 6, but in particular $j$, $k \geq 0$ as the lower limit as shown by dotted line in Fig. 6 holds.

iii) There exists a maximum transit time $\tau_{o,q,\max}(k)$ and thus $\tau_{o,q}(k) \leq \tau_{o,q,\max}(k)$ as the upper limit (grey dotted line in Fig. 6).

The optimal allocation of all sample points in the vector $\mathbf{s}_q^Q(j)$ of the source to the vector $\mathbf{s}_o^O(k)$ of the sink corresponds to the optimal path $\omega_o^q(k)$ through the trellis, taking into account the previously defined conditions i) to iii). To find this path, each individual mapping between the source $q$ and the sink o is represented by a state $\sigma_{o,k}^{q,j}$, and evaluated by a cost value or distance $d_{o,q}(t_j, t)$. measure the square distance

$$d_{o,q}(j,k) = \sqrt{\sum_{e=1}^{E} \left( s_{q,e}^Q(j) - s_{o,e}^O(k) \right)^2} \qquad (6)$$

may be used - but also any other distance measure. Thus, an optimal alignment between source and sink sample points is determined for all properties in parallel. The sole requirements for the distance measure used are

$d_{o,q}(j, k) \geq 0$, the distance measure is unsigned
$d_{o,q}(j, k) \in [0; \infty[$, the distance measure is limited

For the method, the distance measure can also be purely negative, in which case some inequality signs would reverse in the following representation.

The optimal path through the trellis diagram then provides the least total distance, i.e. the sequence of states $\sigma_{o,k}^{q,j}$, for which the following equation holds:

$$\sum_{k=1}^{K} d_{o,q}(j,k) \to \min \qquad (7)$$

To find the optimal path, the following iterative method is used. This uses a matrix $\Delta_o^q$, with entries $\delta_o^q(j,k)$ containing the accumulated distance (also referred to as cost) for each assignment between the measurement points $\mathbf{s}_q^Q(j)$ of the source and $\mathbf{s}_o^O(k)$ of the sink; an auxiliary variable $\mu_o^q(j,k)$, which contains the current path assignment, as well as $\xi$ the upper and $\gamma$ the lower limit from the above restriction.

At first, initialization is defined by the following equations:

$$\delta_o^q(j,k) = \infty \text{ for all } j, k, \qquad (8)$$

$$\delta_o^q(1,1) = d_{o,q}(1,1) \qquad (9)$$

$$\mu_o^q(1,1) = 0. \qquad (10)$$

Secondly, iterations (k> 1) are to be performed with the following restrictions:

$$\delta_o^q(j,k) = \min_{\xi \leq \lambda \leq \gamma} \delta_o^q(\lambda, k-1) + d_{o,q}(j,k) \text{ with } \xi \leq j \leq \gamma, \qquad (11)$$

$$\mu_o^q(j,k) = \operatorname*{argmin}_{\xi \leq \lambda \leq \gamma} \delta_o^q(\lambda, j-1) + d_{o,q}(j,k) \text{ with } \xi \leq j \leq \gamma, \qquad (12)$$

wherein for the respective upper and lower limit applies:

$$\xi = \min\big(T, \max\big(1, k - \tau_{o,q,\max}(k) + 1\big)\big) \text{ and } \gamma = \min\big(N, \max\big(1, k - \tau_{o,q,\min}(k) + 1\big)\big) \qquad (13)$$

Thirdly, a condition is defined for the end of iterations ($t = T$)

$$\omega_o^q(T) = \underset{\xi \le \lambda \le \gamma}{\operatorname{argmin}} \, \delta_o^q(\lambda, T) \ \text{ and } \qquad (14)$$

wherein $\xi, \vartheta$ are defined according to equation (13).

In the fourth place, determination of the optimal path ($K > k \ge 1$) is provided by the following

$$\omega_o^q(k) = \mu_o^q\big(\omega_o^q(k + 1), k + 1\big) \qquad (15)$$

If the process according to equations (8) to (15) is carried out for a sink $o$ and all sources $q$ contributing to this sink, an optimal state sequence will be obtained for each source, from which the sought transit times $\tau_{o,q}(k)$ in equation 4 can be derived by applying equation (5).

Hereinafter, the fractions $\alpha_{o,q}(t)$ of the respective source $q$ for mixing the gas at the sink $o$ are determined

Using the finite state machine of Fig. 5 and the method of equations (8) through (15), the transit times $\tau_{o,q}(k)$ of the gases from the source $q$ to a sink $o$ are determined. This can be applied equally to all sources and sink pairings for which data is available.

However, the fractions $\alpha_{o,q}(k)$ are still unknown. To estimate the fractions, the transit times from only a single source $q$ to a sink $o$ are used to form a fictitious estimate $\tilde{s}_o^{q,O}(k)$ for each sampling point at the sink $o$. For this, equation (4) is modified and simplified

$$\tilde{s}_o^{q,O}(k) = s_q^{Q}\big(k - \tau_{o,q}(k)\big). \qquad (16)$$

Thus, for each source $q$, a mapping to the sink is determined.

There may be time periods in which a source $q$ corresponds to the sink $o$ well (namely, if the source is predominantly or exclusively involved in the formation of the mixed gas) and other time periods wherein the estimated signal does not correspond to the measured signal (if the source is hardly or not at all involved in the formation of the mixed gas).

To quantify the quality of the mapping, for each source $q$ and each time index $k$, the time-dependent amount of the relative error $\epsilon_o^{q,O}(k)$ between the estimated data point $\tilde{s}_o^{q,O}(k)$ and the sample point $s_o^{O}(t)$ at the sink o is determined to:

$$\epsilon_o^{q,O}(k) = \frac{\big\|\tilde{s}_o^{q,O}(k) - s_o^{O}(k)\big\|}{\big\|s_o^{O}(k)\big\|}, 1 \le k \le K \text{ and } s_o^{O}(k) > 0. \qquad (17)$$

Subsequently, the fraction $\alpha_{o,q}(k)$ is calculated from the error: the fraction is reciprocal to the error $\epsilon_o^{q,O}(k)$ from equation (17). For example, the fractions can be calculated using the natural logarithm:

$$\alpha_{o,q}(k) = \frac{\log\Big(\epsilon_o^{q,O}(k)\Big/\sum_{q=1}^{Q}\epsilon_o^{q,O}(k)\Big)}{\sum_{q=1}^{Q}\log\Big(\epsilon_o^{q,O}(k)\Big/\sum_{q=1}^{Q}\epsilon_o^{q,O}(k)\Big)}, 1 \le t \le T \qquad (18)$$

If a determination of the fractions according to equation (18) is made, each source $q$ always and at any time contributes to the formation of the mixed gas, since the method presented here always determines complete and optimal solutions for all input data and for each sample point.

However, usually, only a few sources are involved in the formation of mixed gases. Therefore, an additional bias $b_{o,q}(k)$, which holds for each source $q$ and every time $k$, is introduced. It takes the values "one" or "zero" and determines whether a particular source has contributed to the mix for a given time.

Thus, a possible realization of $b_{o,q}(k)$ can be given as:

$$b_{o,q}(k) = \begin{cases} 1, & \text{for } \epsilon_o^{q,O}(k) < \beta \\ 0, & \text{otherwise} \end{cases} \qquad (19)$$

For determining the threshold $\beta$, e.g. the aforesaid relative error $\epsilon_o^{q,O}(k)$ is taken from equation (17): Normally, measured values are subject to the traffic error limit of 2%, i.e. a measured value deviates normally by no more than 2% of the true value. If the amount of the determined deviation in equation (17) is greater than 2%, it can be assumed that the respective source is not involved in the formation of the mixed gas. In this example $\beta = 0.02$ holds.

Taking the bias $b_{o,q}(k)$ into account, the fraction $\alpha_{o,q}(k)$ yields

$$\alpha_{o,q}(k) = \frac{\log\left(\left(\epsilon_o^{q,O}(k) \cdot b_{o,q}(k)\right) \Big/ \sum_{q=1}^{Q} \epsilon_o^{q,O}(k) \cdot b_{o,q}(k)\right)}{\sum_{q=1}^{Q} \log\left(\left(\epsilon_o^{q,O}(k) \cdot b_{o,q}(k)\right) \Big/ \sum_{q=1}^{Q} \epsilon_o^{q,O}(k) \cdot b_{o,q}(k)\right)}, 1 \leq k \leq K$$

The results of the new method will be presented below and the method is summarized.

The method as defined above was implemented as a prototype and applied to data collected in a real gas or fluid network. This has three natural gas feeds and one bio natural gas feed. At all feed points, various gas properties (calorific value, standard density, $CO_2$) were measured every hour. In addition, the same gas species were measured hourly calibrated at one of the 42 exit points using a mobile PGC.

The transit times $\tau_{o,q}(k)$ were determined using the finite state machine and equations (8) to (15), the fractions $\alpha_{o,q}(k)$ for forming the mixed gas were estimated according to equation (20). Only the $CO_2$ content was used to determine the transit times and fractions.

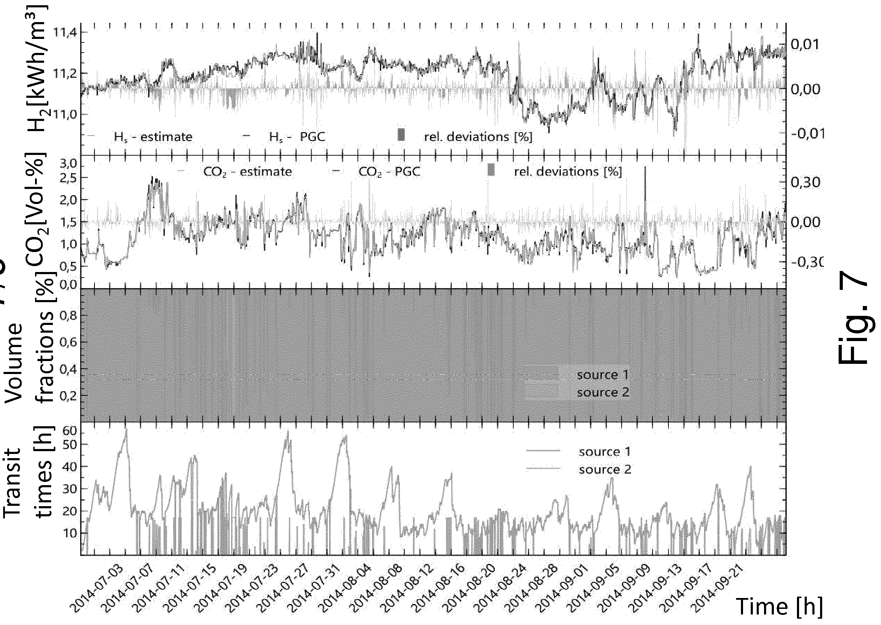

The calorific value was then calculated using the transit times and fractions determined from the $CO_2$ fractions according to equation (4). Both the calculated calorific value and the measured calorific value are shown in Fig. 7. The same Fig. 7 also shows the measured and calculated fractions together with the transit times.

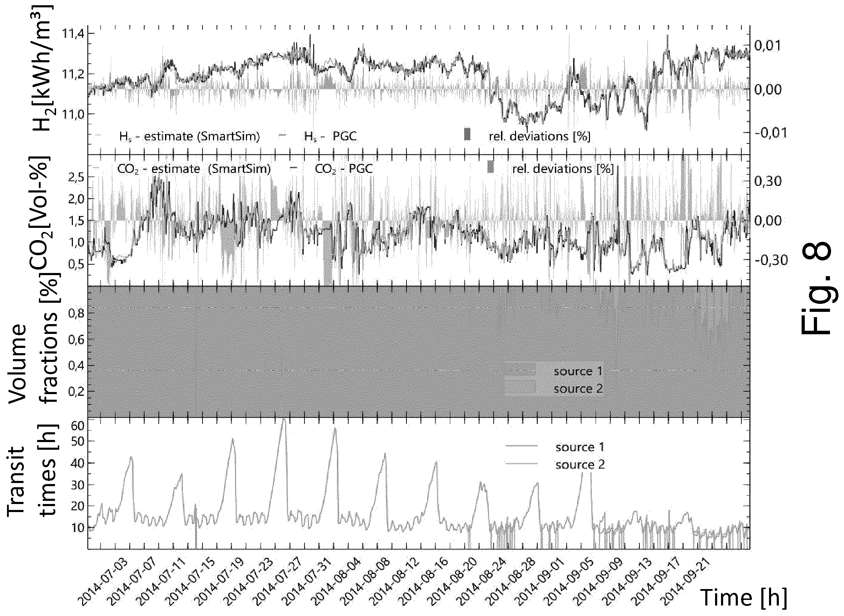

The results of the method presented herein are also compared with prior art. For this purpose, the SmartSim method was used in the same network. The results are shown in Fig. 8. As a qualitative comparison of the graphs from Fig. 7 and Fig. 8 shows, both methods give comparable results - the deviation over several months is significantly lower in the new method than in the prior art. It should also be emphasized that the new method does not require any information about the topology data or even the input and feed-out quantities.

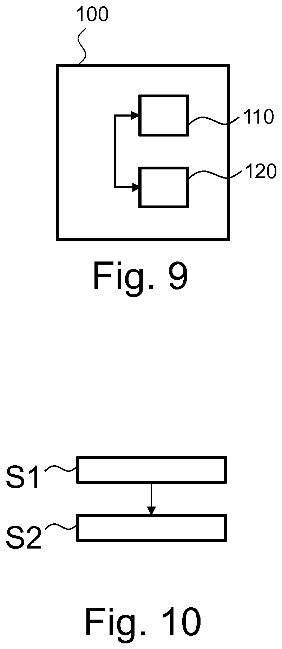

Fig. 9 shows a schematic diagram of an apparatus for estimating transit times $\tau_{o,q}(t)$ of fluids and fractions $\alpha_{o,q}(t)$ of the fluids according to an exemplary embodiment of the invention.

The apparatus comprises at least one sensor element 110 operated to provide sampled upstream data $s_{q,e}^{Q}(t)$ related to a number $E \geq 1$ of fluid properties $e = 1,2, ... ,E$ from the fluids of said first number $Q$ of the upstream network nodes $q = 1,2, ... , Q$ and at least one sensor element operated to provide sampled downstream data $s_{o,e}^{O}(t)$ related to said number $E$ of the fluid properties $e$ from the fluids of at least one downstream network node of said second number $O$ of the downstream network nodes $o$; and

The apparatus comprises a processor 120 configured to iteratively utilize a finite state machine for the alignment between individual measurements of said sampled upstream data $s_{q,e}^{Q}(t)$, derived by sampling upstream network nodes $q$ at discrete points in time $t$ to related individual points in time $t + \tau_{o,q}(t)$ of said sampled downstream data $s_{o,e}^{O}(t)$ related to the identical fluid properties $e$, derived by sampling at least one of the downstream network nodes of said number $O$.

Fig. 10 shows a schematic diagram of a method for estimating transit times $\tau_{o,q}(t)$ of fluids and fractions $\alpha_{o,q}(t)$ of the fluids according to an exemplary embodiment of the invention.

The method comprises the steps of using S1 at least one sensor element operated to provide sampled upstream data $s_{q,e}^{Q}(t)$ related to a number $E \geq 1$ of fluid properties $e = 1,2, ... ,E$ from the fluids of said first number $Q$ of the upstream network nodes $q$, and at least one sensor element operated to provide sampled downstream data $s^{\mathrm{O}}_{o,e}(t)$ related to said number $E$ of the fluid properties e from the fluids of at least one downstream network node of said second number $O$ of the downstream network nodes $o$,

The method comprises the steps of using S2 iteratively utilizing a finite state machine for the alignment between individual measurements of said sampled upstream data $s^{\mathrm{Q}}_{q,e}(t)$, derived by sampling upstream network nodes $q$ at discrete points in time $t$ to related individual points in time $t + \tau_{o,q}$ of said sampled downstream data $s^{\mathrm{O}}_{o,e}(t)$ related to the fluid properties $e$, derived by sampling at least one of the downstream network nodes of said number $O$.

A method for the assignment of gas species is provided by the present invention. In this case, sample points at a sink, which are produced by additive superimposition (that is to say intermixing) from sample points of one or more sources, are assigned to the sources with regard to the transit time and the components. Unlike existing methods, in which the assignment takes place after calculation of the flow-dynamic state of the gas or fluid network taking into account a large number of injection rates, according to the new method the assignment is realized solely by methods of signal processing.

The proposed method of the present invention is provided by using a finite state machine that compensates for the strong time variance of the transmission system. Through an efficient search procedure, those allocations between sample points at the sink and sample points at the source are found from all possible assignments which represent an optimum with respect to a distance measure. From this assignment, the transit times of each source are determined to the considered sink.

According to a further exemplary embodiment of the present invention, a data carrier or a data storage medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims.

However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

**Claims**

1. Method for estimation of

- transit times $\tau_{o,q}(t)$ of fluids between a first number $Q \geq 1$ of upstream network nodes $q = 1,2,...,Q$, interconnected with a second number $O \geq 1$ of downstream network nodes $o = 1,2,...,O$, and corresponding
- fractions $\alpha_{o,q}(t)$ of the fluids, sampled at each upstream network node of said first number $Q$ of the upstream network nodes, composing the fluids sampled at each downstream node of said second number $O$ of the downstream network nodes,
in at least one part of a fluid distribution network;
the method performed by an apparatus and the method comprising the steps of:

- using at least one sensor element operated to provide sampled upstream data $s^{\mathrm{Q}}_{q,e}(t)$ related to a number $E \geq 1$ of fluid properties $e = 1,2,...,E$ from the fluids of said first number $Q$ of the upstream network nodes $q$, and at least one sensor element operated to provide sampled downstream data $s_{o,e}^{O}(t)$ related to said number $E$ of the fluid properties $e$ from the fluids of at least one downstream network node of said second number $O$ of the downstream network nodes $o$,

- iteratively utilizing a finite state machine for the alignment between individual measurements of said sampled upstream data $s_{q,e}^{Q}(t)$, derived by sampling upstream network nodes q at discrete points in time $t$ to related individual points in time $t + \tau_{o,q}$ of said sampled downstream data $s_{o,e}^{O}(t)$ related to the identical fluid properties e, derived by sampling
at least one of the downstream network nodes of said number $O$
- determining transit times $\tau_{o,q}(t)$ of the fluids from a source $q$ to a downstream network node $o$;
- calculating the fraction $\alpha_{o,q}(t)$ from an error between estimated data points and sample points at the downstream network node $o$.

2.  Method according to claim 1,
    wherein the method further comprises the step of utilizing said finite state machine to minimize a total error sum of alignment errors along a path $\omega$ defined by pairings of time indexes $(t, t + \tau_{o,q}(t))$ corresponding to states $\sigma(t, t + \tau_{o,q}(t))$ of individual causal alignments of said sampled upstream data $s_{q,e}^{Q}(t)$ at sample time $t$ and downstream data $s_{o,e}^{O}(t + \tau_{o,q}(t))$ at sample time $t + \tau_{o,q}[t]$, wherein aligned upstream and downstream node data are related to at least one of said number $E$ of fluid properties $e$.

3.  Method according to claim 1 or 2,
    wherein the fluid properties $e$ comprise at least one of the following:
    a concentration of countable fluid particles, a concentration of hydrogen, a concentration of carbon dioxide, a concentration of methane, a concentration of nitrogen, a concentration of argon, a concentration of water, a concentration of helium, a concentration of ethane, a concentration of propane, a concentration of butane, a concentration of carbon oxide, a concentration of an odorant, a concentration of an alkane, a concentration of an alkene, a concentration of oxygen, a concentration of hydrocarbons, a concentration of sulfur, a concentration of hydrogen sulfide, a calorific value, a normal density, a Wobbe index, and combinations thereof.

4.  Method according to any one of the preceding claims,
    wherein said sampled downstream data $s_{o,e}^{O}(t)$ related to the $E \geq 1$ fluid properties is derived through sampling of fluids at successive points in time at at least one downstream node o and said sampled upstream data $s_{q,e}^{Q}(t)$ related to the $E \geq 1$ fluid properties is derived through sampling of fluids at successive points in time at at least one of said upstream network nodes $q$ contributing to the gas composition at the at least one downstream network node of the downstream network nodes o.

5.  Method according to any one of the preceding claims,
    wherein said sampled downstream data $s_{o,e}^{O}(t)$ and/or said sampled upstream data $s_{q,e}^{Q}(t)$ related to the $E \geq 1$ fluid properties is derived through sampling of fluids with constant or varying sampling frequency at any sensor location in the fluid distribution network.

6.  Method according to any one of the preceding claims,
    wherein physical constraints, expressed as a global lower bound transit time $\tau_{o,q,min}$ and a global upper bound transit time $\tau_{o,q,max}$, with $\tau_{o,q,min} \leq \tau_{o,q}(t) \leq \tau_{o,q,max}$, are taken into account for the estimation of the transit times $\tau_{o,q}(t)$ and the fractions $\alpha_{o,q}(t)$, for at least one specific interconnected pair of nodes $o,q$.

7.  Method according to claim 6,
    wherein the global lower bound transit times $\tau_{o,q,min}$ and the global upper bound transit times $\tau_{o,q,max}$ are defined individually for all, with the method according to claim 1, processed interconnected pairs of nodes $o,q$.

8.  Method according to claim 6 or 7,

wherein the global lower bound transit times $\tau_{o,q,\min}$ and the global upper bound transit times $\tau_{o,q,\max}$ are determined for at least one specific interconnected pair of nodes $o,q$ through optimization.

9. Method according to any one of the preceding claims,
wherein local lower bound transit times $\tau_{o,q,\min}(t)$ and local upper bound transit times $\tau_{o,q,\max}(t)$, with $\tau_{o,q,\min}(t) \leq \tau_{o,q}(t) \leq \tau_{o,q,\max}(t)$, are defined for at least one specific interconnected pair of nodes $o,q$, wherein the local lower bound transit times $\tau_{o,q,\min}(t)$ and the local upper bound transit times $\tau_{o,q,\max}(t)$ are preferably defined for all processed specific interconnected pairs of nodes $o,q$.

10. Method according to claim 9, wherein the local lower bound transit times $\tau_{o,q,\min}(t)$ and the local upper bound transit times $\tau_{o,q,\max}(t)$ are determined for a at least one specific interconnected pair of nodes $o,q$ through optimization.

11. Method according to any one of the preceding claims 9 to 10,
wherein the optimization is performed using at least one similarity measure between the downstream data and the upstream data.

12. Method according to any one of the preceding claims,
wherein said sampled upstream data $s_{q,e}^{Q}(t)$ is sampled at a number of at least $Q \geq 2$ upstream nodes and said sampled downstream data $s_{o,e}^{O}(t)$ is sampled at a number of least $O \geq 1$ network downstream nodes.

13. Method according to any one of the preceding claims,
wherein said sampled downstream data $s_{o,e}^{O}(t)$ and/or said sampled upstream data $s_{q,e}^{Q}(t)s$ related to $E \geq 2$ fluid properties, wherein at least one of the $E$ fluid properties is processed with said finite state machine.

14. Method according to any one of the preceding claims,
wherein the fluid transported in the distribution network is combustible gas and the fluid distribution network denotes gas distribution networks.

15. An apparatus for estimating transit times $\tau_{o,q}(t)$ of fluids between a first number $Q \geq 1$ of upstream network nodes $q = 1,2,...,Q$, interconnected with a second number $O \geq 1$ of downstream network nodes $o = 1,2,...,O$, and for estimating corresponding fractions $\alpha_{o,q}(t)$ of the fluids, sampled at each upstream network node of said first number $Q$ of the upstream network nodes, composing the fluids sampled at each downstream node of said second number $O$ of the downstream network nodes, in at least one part of a fluid distribution network, the apparatus comprising

- at least one sensor element operated to provide sampled upstream data $s_{q,e}^{Q}(t)$ related to a number $E \geq 1$ of fluid properties $e = 1,2,...,E$ from the fluids of said first number $Q$ of the upstream network nodes $q = 1,2,...,Q$ and at least one sensor element operated to provide sampled downstream data $s_{o,e}^{O}(t)$ related to said number $E$ of the fluid properties $e$ from the fluids of at least one downstream network node of said second number $O$ of the downstream network nodes $o$; and
- a processor configured to iteratively utilize a finite state machine for the alignment between individual measurements of said sampled upstream data $s_{q,e}^{Q}(t)$, derived by sampling upstream network nodes $q$ at discrete points in time t to related individual points in time $t + \tau_{o,q}(t)$ of said sampled downstream data $s_{o,e}^{O}(t)$ related to the identical fluid properties e, derived by sampling at least one of the downstream network nodes of said number $O$.
- the processor being further configured to determine transit times $\tau_{o,q}(t)$ of the fluids from a source q to a downstream network node o;
- the processor being further configured to calculate the fraction $\alpha_{o,q}(t)$ from an error between estimated data points and sample points at the downstream network node o.

**Patentansprüche**

1. Verfahren zur Schätzung von

   - Durchlaufzeiten $\tau_{o,q}(t)$ von Fluiden zwischen einer ersten Anzahl $Q \geq 1$ von stromaufwärts gelegenen Netzwerkknoten $q = 1,2, ... , Q$, die mit einer zweiten Anzahl $O \geq 1$ von stromabwärts gelegenen Netzwerkknoten $o = 1,2, ..., O$ verbunden sind, und entsprechenden
   - Anteilen $\alpha_{o,q}(t)$ der Fluide, die an jedem stromaufwärts gelegenen Netzwerkknoten der ersten Anzahl $Q$ der stromaufwärts gelegenen Netzwerkknoten überprüft wurden, bilden die an jedem stromabwärts gelegenen Knoten der zweiten Anzahl 0 der stromabwärts gelegenen Netzwerkknoten überprüften Flüssigkeiten, in mindestens einem Teil eines Fluidverteilungsnetzes;

   wobei das Verfahren von einer Vorrichtung ausgeführt wird und das Verfahren die folgenden Schritte umfasst:

   - Verwenden mindestens eines Sensorelements, das so betrieben wird, dass es abgetastete stromaufwärtsgerichtete Daten $s_{q,e}^{Q}(t)$ bereitstellt, die sich auf eine Anzahl $E \geq 1$ von Fluideigenschaften $e = 1,2, ..., E$ aus den Fluiden der ersten Anzahl $Q$ der stromaufwärts gelegenen Netzwerkknoten $q$ beziehen, und mindestens eines Sensorelements, das so betrieben wird, dass es abgetastete stromabwärtsgerichtete Daten $s_{o,e}^{O}(t)$ bereitstellt, die sich auf die Anzahl $E$ der Fluideigenschaften $e$ aus den Fluiden mindestens eines stromabwärts gelegenen Netzwerkknotens der zweiten Anzahl $O$ der stromabwärts gelegenen Netzwerkknoten $o$ beziehen,
   - iteratives Verwenden einer finiten Elementemaschine zur Ausrichtung einzelner Messungen der abgetasteten stromaufwärts gelegenen Daten $s_{q,e}^{Q}(t)$, die durch Abtasten stromaufwärts gelegener Netzwerkknoten $q$ zu diskreten Zeitpunkten $t$ gewonnen wurden, auf zugehörige einzelne Zeitpunkte $t + \tau_{o,q}$ der abgetasteten stromabwärts gelegenen Daten $s_{o,e}^{O}(t)$, die sich auf die identischen Fluideigenschaften $e$ beziehen und durch Abtasten mindestens eines der stromabwärts gelegenen Netzwerkknoten der Anzahl $O$ gewonnen wurden
   - Bestimmen von Laufzeiten $\tau_{o,q}(t)$ der Fluide von einer Quelle $q$ zu einem stromabwärts gelegenen Netzwerkknoten $o$;
   - Berechnung des Anteils $\alpha_{o,q}(t)$ aus einem Fehler zwischen geschätzten Datenpunkten und Stichprobenpunkten am nachgelagerten Netzwerkknoten $o$.

2. Verfahren nach Anspruch 1,
   wobei das Verfahren ferner den Schritt umfasst, die finite Elementemaschine zu nutzen, um eine Gesamtfehlersumme von Ausrichtungsfehlern entlang eines Pfades $\omega$ zu minimieren, der durch Paarungen von Zeitindizes $(t, t + \tau_{o,q}(t))$ definiert ist, die Zuständen $\sigma(t, t + \tau_{o,q}(t))$ einzelner kausaler Ausrichtungen der abgetasteten Upstream-Daten $s_{q,e}^{Q}(t)$ zum Abtastzeitpunkt $t$ und der Downstream-Daten $s_{o,e}^{O}(t + \tau_{o,q}(t))$ zum Abtastzeitpunkt $t + \tau_{o,q}[t]$ entsprechen, wobei die ausgerichteten Upstream- und Downstream-Knotendaten mit mindestens einer der Anzahl $E$ von Fluideigenschaften $e$ in Zusammenhang stehen.

3. Verfahren nach Anspruch 1 oder 2,
   wobei die Fluideigenschaften e mindestens eines der folgenden Elemente umfassen: eine Konzentration zählbarer Fluidpartikel, eine Wasserstoffkonzentration, eine Kohlendioxidkonzentration, eine Methankonzentration, eine Stickstoffkonzentration, eine Argonkonzentration, eine Wasserkonzentration, eine Heliumkonzentration, eine Ethan-Konzentration, eine Propan-Konzentration, eine Butan-Konzentration, eine Kohlenmonoxid-Konzentration, eine Konzentration eines Geruchsstoffs, eine Konzentration eines Alkans, eine Konzentration eines Alkens, eine Konzentration von Sauerstoff, eine Konzentration von Kohlenwasserstoffen, eine Konzentration von Schwefel, eine Konzentration von Schwefelwasserstoff, einen Brennwert, eine Normaldichte, einen Wobbe-Index und Kombinationen davon.

4. Verfahren gemäß einem der vorstehenden Ansprüche,

   wobei die abgetasteten stromabwärts gelegenen Daten $s_{o,e}^{O}(t)$, die sich auf die Eigenschaften der Fluide $E \geq 1$ beziehen, durch Abtastung von Fluiden zu aufeinanderfolgenden Zeitpunkten an mindestens einem stromabwärts gelegenen Knoten *o* abgeleitet werden und die abgetasteten stromaufwärts gelegenen Daten $s_{q,e}^{Q}(t)$, die sich auf die Eigenschaften der Fluide *E* ≥ 1 beziehen, durch Abtastung von Fluiden zu aufeinanderfolgenden Zeitpunkten an mindestens einem der stromaufwärts gelegenen Netzwerkknoten *q* abgeleitet werden, die zur Gaszusammensetzung an dem mindestens einen stromabwärts gelegenen Netzwerkknoten der stromabwärts gelegenen Netzwerkknoten *o* beitragen.

5. Verfahren gemäß einem der vorstehenden Ansprüche,

wobei die abgetasteten stromabwärts gelegenen Daten $s_{o,e}^{O}(t)$ und/oder die abgetasteten stromaufwärts gelegenen Daten $s_{q,e}^{Q}(t)$, die sich auf die Fluideigenschaften *E* ≥ 1 beziehen, durch Abtastung von Fluiden mit konstanter oder variierender Abtastfrequenz an einem beliebigen Sensorstandort im Fluidverteilungsnetzwerk abgeleitet werden.

6. Verfahren nach einem der vorstehenden Ansprüche,
wobei physikalische Einschränkungen, ausgedrückt als eine globale untere Grenze der Laufzeit $\tau_{o,q,min}$ und eine globale obere Grenze der Laufzeit $\tau_{o,q,max}$, mit $\tau_{o,q,min} \leq \tau_{o,q}(t) \leq \tau_{o,q,max}$, bei der Schätzung der Laufzeiten $\tau_{o,q}(t)$ und der Anteile $\alpha_{o,q}(t)$ für mindestens ein spezifisches, miteinander verbundenes Knotenpaar *o,q* berücksichtigt werden.

7. Verfahren nach Anspruch 6,
wobei die globalen unteren Grenzwerte für die Transitzeiten $\tau_{o,q,min}$ und die globalen oberen Grenzwerte für die Transitzeiten $\tau_{o,q,max}$ für alle mit dem Verfahren nach Anspruch 1 verarbeiteten miteinander verbundenen Knotenpaare *o,q* individuell definiert werden.

8. Verfahren nach Anspruch 6 oder 7,
wobei die globalen unteren Grenzwerte für die Laufzeiten $\tau_{o,q,min}$ und die globalen oberen Grenzwerte für die Laufzeiten $\tau_{o,q,max}$ für mindestens ein spezifisches miteinander verbundenes Knotenpaar *o,q* durch Optimierung bestimmt werden.

9. Verfahren gemäß einem der vorstehenden Ansprüche,
wobei lokale untere Grenzwerte für die Übertragungszeiten $\tau_{o,q,min}(t)$ und lokale obere Grenzwerte für die Übertragungszeiten $\tau_{o,q,max}(t)$, mit $\tau_{o,q,min}(t) \leq \tau_{o,q}(t) \leq \tau_{o,q,max}(t)$, für mindestens ein spezifisches, miteinander verbundenes Knotenpaar *o,q* definiert sind, wobei die lokalen unteren Grenzwerte für die Übertragungszeiten $\tau_{o,q,min}(t)$ und die lokalen oberen Grenzwerte für die Übertragungszeiten $\tau_{o,q,max}(t)$ vorzugsweise für alle verarbeiteten spezifischen, miteinander verbundenen Knotenpaare *o,q* definiert sind.

10. Verfahren nach Anspruch 9, wobei die lokalen unteren Grenzwerte für die Laufzeiten $\tau_{o,q,min}(t)$ und die lokalen oberen Grenzwerte für die Laufzeiten $\tau_{o,q,max}(t)$ für mindestens ein spezifisches, miteinander verbundenes Knotenpaar *o,q* durch Optimierung bestimmt werden.

11. Verfahren nach einem der vorstehenden Ansprüche 9 bis 10,
wobei die Optimierung unter Verwendung mindestens eines Ähnlichkeitsmaßes zwischen den Downstream-Daten und den Upstream-Daten durchgeführt wird.

12. Verfahren nach einem der vorstehenden Ansprüche,

wobei die abgetasteten Upstream-Daten $s_{q,e}^{Q}(t)$ an mindestens *Q* ≥ 2 Upstream-Knoten abgetastet werden und die abgetasteten Downstream-Daten $s_{o,e}^{O}(t)$ an mindestens 0 ≥ 1 Netzwerk-Downstream-Knoten abgetastet werden.

13. Verfahren gemäß einem der vorstehenden Ansprüche,

wobei die abgetasteten Downstream-Daten $s_{o,e}^{O}(t)$ und/oder die abgetasteten Upstream-Daten $s_{q,e}^{Q}(t)$ sich auf *E* ≥ 2 Fluideigenschaften beziehen, wobei mindestens eine der E Fluideigenschaften mit der endlichen Zustandsmaschine verarbeitet wird.

14. Verfahren gemäß einem der vorstehenden Ansprüche,

wobei das im Verteilungsnetz transportierte Fluid brennbares Gas ist und das Fluidverteilungsnetz Gasverteilungsnetze bezeichnet.

**15.** Vorrichtung zur Schätzung von Durchlaufzeiten $\tau_{o,q}(t)$ von Fluiden zwischen einer ersten Anzahl $Q \geq 1$ von strom-aufwärts gelegenen Netzwerkknoten $q = 1,2, ... ,Q$, die mit einer zweiten Anzahl $0 \geq 1$ von stromabwärts gelegenen Netzwerkknoten $o = 1,2, ..., O$ verbunden sind, und zur Schätzung entsprechender Anteile $\alpha_{o,q}(t)$ der Fluide, die an jedem stromaufwärts gelegenen Netzwerkknoten der ersten Anzahl Q der stromaufwärts gelegenen Netzwerkknoten entnommen wurden und die die an jedem stromabwärts gelegenen Knoten der zweiten Anzahl 0 der stromabwärts gelegenen Netzwerkknoten entnommenen Fluide bilden, in mindestens einem Teil eines Fluidverteilungsnetzes, wobei die Vorrichtung umfasst

- mindestens ein Sensorelement, das so betrieben wird, dass es erfasste stromaufwärts gelegene Daten liefert $s_{q,e}^{Q}(t)$ in Bezug auf eine Anzahl $E \geq 1$ von Fluideigenschaften e = 1,2, ...,E aus den Fluiden der ersten AnzahlQ der stromaufwärts gelegenen Netzwerkknoten $q = 1,2, ..., Q$ und mindestens ein Sensorelement, das so betrieben wird, dass es abgetastete stromabwärts gelegene Daten $s_{o,e}^{O}(t)$ in Bezug auf die Anzahl E der Fluideigenschaften e aus den Fluiden mindestens eines stromabwärts gelegenen Netzwerkknotens der zweiten Anzahl 0 der stromabwärts gelegenen Netzwerkknoteno bereitstellt; und

- $s_{o,e}^{O}(t)$ einen Prozessor, der so konfiguriert ist, dass er iterativ eine endliche Zustandsmaschine nutzt, um einzelne Messungen der abgetasteten stromaufwärts gelegenen Daten $s_{q,e}^{Q}(t)$, die durch Abtastung strom-aufwärts gelegener Netzwerkknotenq zu diskreten Zeitpunktent gewonnen wurden, mit entsprechenden ein-zelnen Zeitpunkten $t + \tau_{o,q}(t)$ der abgetasteten stromabwärts gelegenen Daten , die sich auf die identischen Fluideigenschaftene beziehen und durch Abtastung mindestens eines der stromabwärts gelegenen Netzwerkknoten der genannten Anzahl 0
- wobei der Prozessor ferner so konfiguriert ist, dass er die Laufzeiten $\tau_{o,q}(t)$ der Fluide von einer Quelle q zu einem nachgelagerten Netzwerkknoten o bestimmt;
- wobei der Prozessor ferner so konfiguriert ist, dass er den Anteil $\alpha_{o,q}(t)$ aus einer Abweichung zwischen geschätzten Datenpunkten und Abtastpunkten am stromabwärts gelegenen Netzwerkknoten o berechnet.

## Revendications

**1.** Procédé d'estimation de :

- temps de transit $\tau_{o,q}(t)$ de fluides entre un premier nombre $Q \geq 1$ de nœuds de réseau amont $q = 1,2,...,Q$, interconnectés avec un deuxième nombre $O \geq 1$ de nœuds de réseau aval $o = 1,2,...,O$, et
- fractions correspondantes $\alpha_{o,q}(t)$ des fluides, échantillonnées au niveau de chaque nœud de réseau amont dudit premier nombre Q de nœuds de réseau amont, composant les fluides échantillonnés au niveau de chaque nœud aval dudit deuxième nombre O de nœuds de réseau aval, dans au moins une partie d'un réseau de distribution de fluide ; le procédé étant effectué par un appareil et le procédé comprenant les étapes consistant à :

- utiliser au moins un élément capteur exploité afin de fournir des données amont échantillonnées $s_{q,e}^{Q}(t)$ liées à un nombre $E \geq 1$ de propriétés de fluide e = 1,2,...,E provenant des fluides dudit premier nombre Q des nœuds de réseau amont q , et au moins un élément capteur exploité afin de fournir des données aval échantillonnées $s_{o,e}^{O}(t)$ liées audit nombre E des propriétés de fluide e provenant des fluides d'au moins un nœud de réseau aval dudit deuxième nombre O des nœuds de réseau aval o ,
- utiliser de manière itérative une machine à états finis pour l'alignement entre des mesures individuelles desdites données amont échantillonnées $s_{q,e}^{Q}(t)$, obtenues par échantillonnage de nœuds de réseau amont q à des points discrets dans le temps t , avec des points individuels liés dans le temps $t + \tau_{o,q}$ desdites données aval échantillonnées $s_{o,e}^{O}(t)$ liées aux propriétés de fluide identiques e, obtenues par échantillonnage d'au moins un des nœuds de réseau aval dudit nombre *O*,
- déterminer des temps de transit $\tau_{o,q}$(t) des fluides depuis une source *q* jusqu'à un nœud de réseau aval *o* ;
- calculer la fraction $\alpha_{o,q}$(t) à partir d'une erreur entre des points de données estimés et des points échantillonnés au niveau du nœud de réseau aval o.

**2.** Procédé selon la revendication 1,
dans lequel le procédé comprend en outre l'étape consistant à utiliser ladite machine à états finis afin de minimiser une somme d'erreurs totale d'erreurs d'alignement le long d'un chemin $\omega$ défini par des appariements d'indices temporels (t, t + $\tau_{o,q}$(t)) correspondant à des états $\alpha$(t, t + $\tau_{o,q}$(t)) d'alignements causaux individuels desdites données amont échantillonnées $s_{q,e}^{Q}(t)$ au temps d'échantillonnage *t* et des données aval $s_{o,e}^{O}(t + \tau_{o,q}(t))$ au temps d'échantillonnage *t* + $\tau_{o'q}$ [t], les données alignées des nœuds amont et aval étant liées à au moins une dudit nombre *E* de propriétés de fluide *e*.

**3.** Procédé selon la revendication 1 ou 2,
dans lequel les propriétés de fluide e comprennent au moins l'une des suivantes :
une concentration en particules fluides dénombrables, une concentration en hydrogène, une concentration en dioxyde de carbone, une concentration en méthane, une concentration en azote, une concentration en argon, une concentration en eau, une concentration en hélium, une concentration en éthane, une concentration en propane, une concentration en butane, une concentration en oxyde de carbone, une concentration en un odorant, une concentration en un alcane, une concentration en un alcène, une concentration en oxygène, une concentration en hydrocarbures, une concentration en soufre, une concentration en sulfure d'hydrogène, un pouvoir calorifique, une densité normale, un indice de Wobbe, et des combinaisons de ceux-ci.

**4.** Procédé selon l'une quelconque des revendications précédentes,
dans lequel lesdites données aval échantillonnées $s_{o,e}^{O}(t)$ liées aux *E* ≥ 1 propriétés de fluide sont obtenues par échantillonnage de fluides à des points successifs dans le temps au niveau d'au moins un nœud aval *o*, et lesdites données amont échantillonnées $s_{q,e}^{Q}(t)$ liées aux *E* ≥ 1 propriétés de fluide sont obtenues par échantillonnage de fluides à des points successifs dans le temps au niveau d'au moins un desdits nœuds de réseau amont *q* contribuant à la composition gazeuse au niveau de l'au moins un nœud de réseau aval parmi les nœuds de réseau aval o.

**5.** Procédé selon l'une quelconque des revendications précédentes,
dans lequel lesdites données aval échantillonnées $s_{o,e}^{O}(t)$ et/ou lesdites données amont échantillonnées $s_{q,e}^{Q}(t)$ liées aux *E* ≥ 1 propriétés de fluide sont obtenues par échantillonnage de fluides avec une fréquence d'échantillonnage constante ou variable à n'importe quel emplacement de capteur dans le réseau de distribution de fluide.

**6.** Procédé selon l'une quelconque des revendications précédentes,
dans lequel des contraintes physiques, exprimées sous la forme d'un temps de transit minimal global $\tau_{o,q,min}$ et d'un temps de transit maximal global $\tau_{o,q,max}$, avec $\tau_{o,q,min} \le \tau_{o,q}(t) \le \tau_{o,q,max}$, sont prises en compte pour l'estimation des temps de transit $\tau_{o,q}(t)$ et des fractions $\alpha_{o,q}(t)$, pour au moins une paire spécifique interconnectée de nœuds *o,q* .

**7.** Procédé selon la revendication 6,
dans lequel les temps de transit minimaux globaux $\tau_{o,q'min}$ et les temps de transit maximaux globaux $\tau_{o,q,max}$ sont définis individuellement pour toutes les paires interconnectées de nœuds *o,q* traitées avec le procédé selon la revendication 1.

**8.** Procédé selon la revendication 6 ou 7,
dans lequel les temps de transit minimaux globaux $\tau_{o,q,min}$ et les temps de transit maximaux globaux $\tau_{o,q,max}$ sont déterminés pour au moins une paire spécifique interconnectée de nœuds o,q par optimisation.

**9.** Procédé selon l'une quelconque des revendications précédentes,
dans lequel des temps de transit minimaux locaux $\tau_{o,q,min}(t)$ et des temps de transit maximaux locaux $\tau_{o,q,max}(t)$, avec $\tau_{o,q,min}(t) \le \tau_{o,q}(t) \le \tau_{o,q,max}(t)$, sont définis pour au moins une paire spécifique interconnectée de nœuds o,q , les temps de transit minimaux locaux $\tau_{o,q,min}(t)$ et les temps de transit maximaux locaux $\tau_{o,q,max}(t)$ étant de préférence définis pour toutes les paires spécifiques interconnectées de nœuds o,q traitées.

**10.** Procédé selon la revendication 9,
dans lequel les temps de transit minimaux locaux $\tau_{o,q,\mathrm{min}}(t)$ et les temps de transit maximaux locaux $\tau_{o,q,\mathrm{max}}(t)$ sont déterminés pour au moins une paire spécifique interconnectée de nœuds o,q par optimisation.

**11.** Procédé selon l'une quelconque des revendications 9 à 10 précédentes, dans lequel l'optimisation est effectuée à l'aide d'au moins une mesure de similarité entre les données aval et les données amont.

**12.** Procédé selon l'une quelconque des revendications précédentes,

dans lequel lesdites données amont échantillonnées $s_{q,e}^{Q}(t)$ sont échantillonnées au niveau d'un nombre d'au

moins $Q \geq 2$ nœuds amont et lesdites données aval échantillonnées $s_{o,e}^{O}(t)$ sont échantillonnées au niveau d'un nombre d'au moins $O \geq 1$ nœuds aval de réseau.

**13.** Procédé selon l'une quelconque des revendications précédentes,

dans lequel lesdites données aval échantillonnées $s_{o,e}^{O}(t)$ et/ou lesdites données amont échantillonnées $s_{q,e}^{Q}(t)$ sont liées à $E \geq 2$ propriétés de fluide, au moins une des $E$ propriétés de fluide étant traitée avec ladite machine à états finis.

**14.** Procédé selon l'une quelconque des revendications précédentes,
dans lequel le fluide transporté dans le réseau de distribution est un gaz combustible et le réseau de distribution de fluide désigne des réseaux de distribution de gaz.

**15.** Appareil destiné à estimer des temps de transit $\tau_{o,q}(t)$ de fluides entre un premier nombre $Q \geq 1$ de nœuds de réseau amont $q = 1,2,...,Q$ interconnectés avec un deuxième nombre $O \geq 1$ de nœuds de réseau aval $o = 1,2,...,O$, et destiné à estimer des fractions correspondantes $\alpha_{o,q}(t)$ des fluides, échantillonnées au niveau de chaque nœud de réseau amont dudit premier nombre $Q$ de nœuds de réseau amont, composant les fluides échantillonnés au niveau de chaque nœud aval dudit deuxième nombre $O$ des nœuds de réseau aval, dans au moins une partie d'un réseau de distribution de fluide, l'appareil comprenant :

- au moins un élément capteur exploité afin de fournir des données amont échantillonnées $s_{q,e}^{Q}(t)$ liées à un nombre $E \geq 1$ de propriétés de fluide $e = 1,2,...,E$ provenant des fluides dudit premier nombre $Q$ de nœuds de réseau amont $q = 1,2,...,Q$ et au moins un élément capteur exploité afin de fournir des données aval échantillonnées $s_{o,e}^{O}(t)$ liées audit nombre $E$ des propriétés de fluide $e$ provenant des fluides d'au moins un nœud de réseau aval dudit deuxième nombre $O$ de nœuds de réseau aval $o$ ; et
- un processeur configuré pour utiliser de manière itérative une machine à états finis pour l'alignement entre des mesures individuelles desdites données amont échantillonnées $s_{q,e}^{Q}(t)$, obtenues par échantillonnage de nœuds de réseau amont $q$ à des points discrets dans le temps $t$, avec des points individuels liés dans le temps t +

$\tau_{o,q}(t)$ desdites données aval échantillonnées $s_{o,e}^{O}(t)$ liées aux propriétés de fluide identiques e, obtenues par échantillonnage d'au moins un des nœuds de réseau aval dudit nombre $O$ ;
- le processeur étant en outre configuré pour déterminer des temps de transit $\tau_{o,q}(t)$ des fluides depuis une source $q$ jusqu'à un nœud de réseau aval $o$ ;
- le processeur étant en outre configuré pour calculer la fraction $\alpha_{o,q}(t)$ à partir d'une erreur entre des points de données estimés et des points échantillonnés au niveau du nœud de réseau aval $o$.

Fig. 1

assumed direction of
data transmission

upstream nodes

downstream node

source 1

sink 1

source 2

source 3

# Fig. 2

Fig. 3

Fig. 4

Fig. 5

Fig. 6

Fig. 7

Fig. 8

100

110

120

Fig. 9

S1

S2

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2450704 B1

**Non-patent literature cited in the description**

- **BENJAMIN HILLER et al.** A system to evaluate gas network capacities: Concepts and implementation. *European Journal of Operational Research*, 01 February 2017, 7 97-808
- **MARTIN GROSS et al.** On the complexity of instationary gas flows. *OPERATIONS RESEARCH LETTERS*, 17 February 2018, vol. 46 (3), 286-290
- **KENNETH L COOKE et al.** The shortest route through a network with time-dependent internodal transit times. *JOURNAL OF MATHEMATICAL ANALYSIS AND APPLICATIONS*, 01 June 1966, vol. 14 (3), 493-498
- **ALTFELD K. ; SCHLEY P.** Entwicklung der Erdgasbeschaffenheiten in Europa. *Fachbericht Gaswärme International*, 2012, 57-63
- DVGW-Arbeitsblatt G 685. *Gasabrechnung*, 2008
- **SCHENK J.** ; **P. SCHLEY** ; **A. HIELSCHER** ; **C. FERNANDEZ** ; **S. MAURER**. Brennwertverfolgung in Verteilnetzen: Teil 2: Auswertung Feldversuch. *gwf-Gas/Erdgas*, 2011, 676-683
- **P. SCHLEY** ; **J. SCHENK** ; **A. HIELSCHER**. Brennwertverfolgung in Verteilnetzen: Teil 1: Entwicklung und Validierung des Verfahrens. *gwf-Gas/Erdgas*, 2011, 552-556
- **SCHENK J.** ; **P. SCHLEY** ; **A. HIELSCHER** ; **C. FERNANDEZ** ; **S. MÄURER**. Brennwertverfolgung in Verteilnetzen: Teil 2: Auswertung Feldversuch. *gwf-Gas/Erdgas*, 2011, 676-683
- **C. FIEBIG** ; **A. HILSCHER** ; **R. SPAN** ; **P. SCHLEY** ; **J. SCHENK**. Gas Quality Tracking in Gas Grids including Gases from Renewable Sources. *Kopenhagen: EGATEC*, 2013